# EUROPEAN PATENT APPLICATION

(11) **EP 3 266 768 A1**
(43) Date of publication of application: **10.01.2018**
(21) Application number: 16760992.4
(22) Date of filing: 29.02.2016
(51) Int. Cl.: C07D 215/14, C07D 295/092, A61K 31/47, A61K 31/496, A61P 25/08

(54) **SELECTIVE INHIBITORS OF HEMICHANNELS FORMED BY CONNEXINS FOR THE TREATMENT OF EPILEPSY**

(30) Priority: 06.03.2015 US 201562129432 P
(71) Applicant: Pontificia Universidad Católica de Chile, Santiago (CL)
(72) Inventor: SAEZ, Juan Carlos, Santiago (CL); LAGOS, Carlos, Santiago (CL); MATURANA, Carola, Santiago (CL)
(74) Representative: Manuel Illescas Asociados, S.L.
(86) International application number: PCT/CL2016/050007
(87) International publication number: WO 2016/141499

(57) **Abstract**

The present invention provides connexin hemichannel blockers for the treatment of epilepsy.

In particular, the present invention relates to organic compounds which act as selective blockers of connexin hemichannels which are useful in the treatment of epilepsy.

In another embodiment of the present invention it is provided a composition comprising selective connexin hemichannels blocker compounds useful in the treatment of epilepsy in combination with other antiepileptic compounds.

## Description

### TECHNICAL FIELD OF THE INVENTION

The present invention provides connexin hemichannels blockers for the treatment of epilepsy.

In particular, the present invention relates to organic compounds that act as selective blockers of connexin hemichannels which are useful in the treatment of epilepsy.

In another embodiment, the present invention provides a composition comprising a selective connexin hemichannel blocker compounds useful in the treatment of epilepsy in combination with other antiepileptic compounds.

### BACKGROUND

Currently, treatments for epilepsy and management of epileptic seizures are focused on drugs that target molecular targets located in neurons.

Current drug therapy allows partial control of seizures in only 70% of cases trough blocking of neuronal sodium ion channels in the brain. These sodium ion channels are the same as those that determine the excitability of the brain and the firing of neuronal electrical responses.

However, in addition to the fact that the compounds currently used are unable to fully control convulsions, such drugs have adverse side effects, such as hearing deterioration and are not efficient in all patients. It may be necessary to change the dose from time to time or that epilepsy may not improve even after having tested two or three anticonvulsant drugs (termed "treatment resistant epilepsy").

For example, carbamazepine, valproate and phenytoin are among the existing drugs useful in the treatment of epilepsy, among others.

Phenytoin or diphenylhydantoin is a compound used for seizure management, which reduces electrical conductivity between neurons by blocking voltage-gated sodium ion channels. It is used to control certain types of seizures and to treat and prevent seizures that may occur during or after surgery of the brain or nervous system. This compound is also used as an anti-arrhythmic agent as it blocks the cardiac sodium ion channels.

According to the state of the art, patent application CL 1556-2014 indicates that although numerous connexins modulators are known and several lines of research suggest that their use may have therapeutic utility in the treatment of epilepsy, cardiac arrhythmias, cancer, stroke or other conditions (Spray, Rozental et al. 2002), most of them are non-specific, affecting the activity not only of the hemichannels but also of intercellular channels formed by connexins, and therefore, the compounds presently known in the art have low efficacy and nonspecific mechanisms of action or secondary to the pharmacological action in other therapeutic targets that modify the activity of connexin hemichannels, and are therefore responsible for side effects that do not allow to use them in chronic schemes nor in doses to effectively control the pathologies of interest.

Based on the above, it is concluded that available hemichannel blockers formed by connexins also inhibit gap junctions, which play an important role in the coordination of numerous electrical and metabolic responses in various tissues. Therefore, it is desirable to obtain selective hemichannel blocking compounds that have no effect on gap junction channels, which could be useful for rationally designing therapeutic treatments for various diseases, minimizing undesired side effects.

### BRIEF DESCRIPTION OF THE DRAWINGS

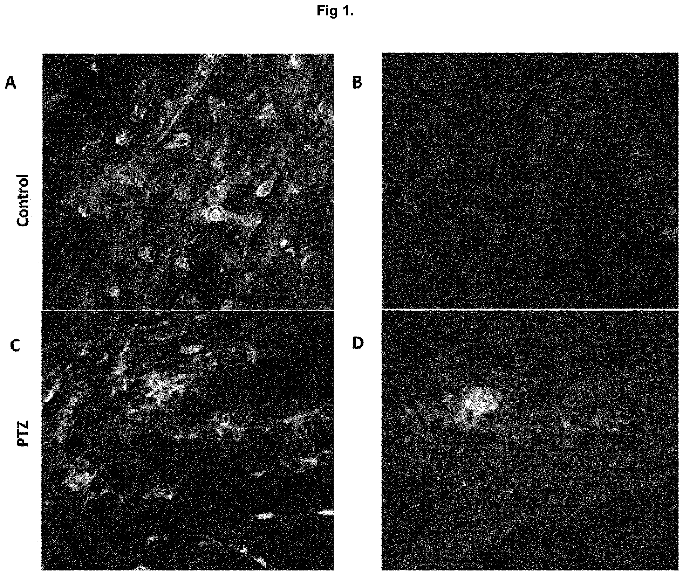

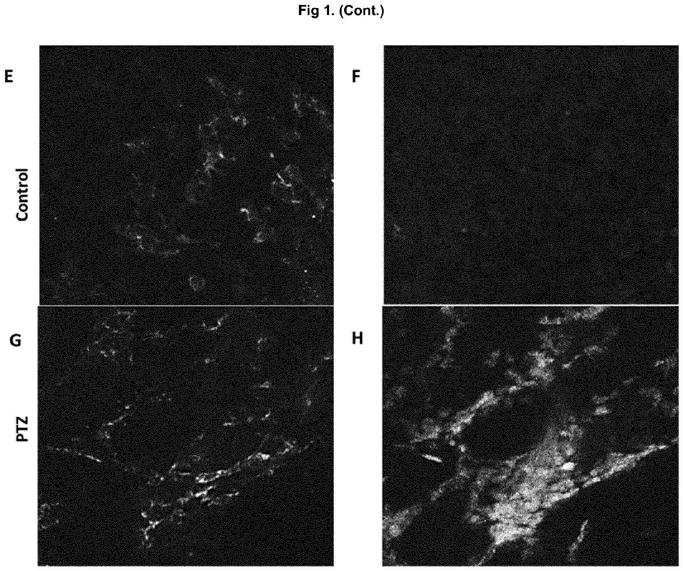

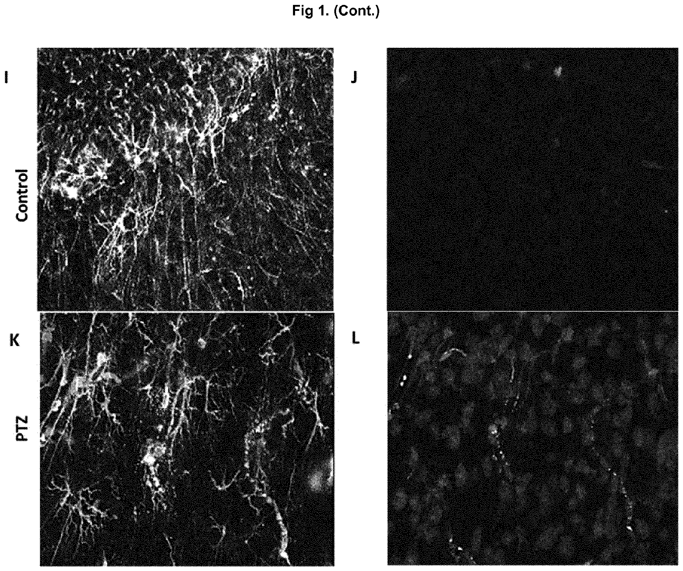

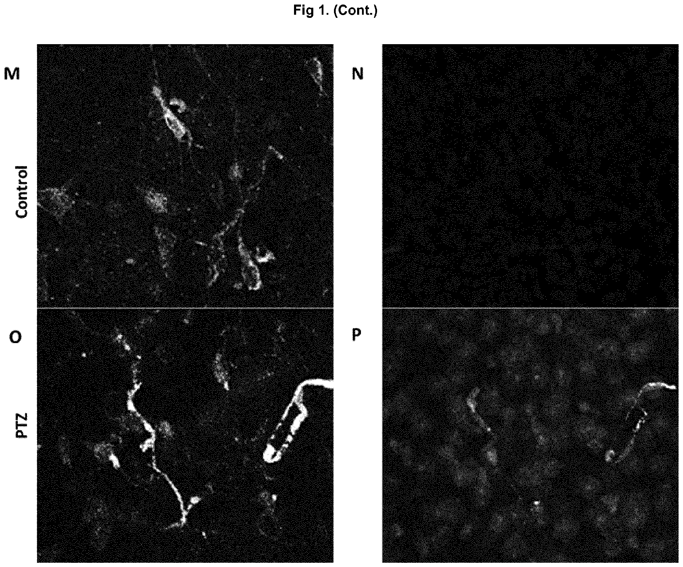

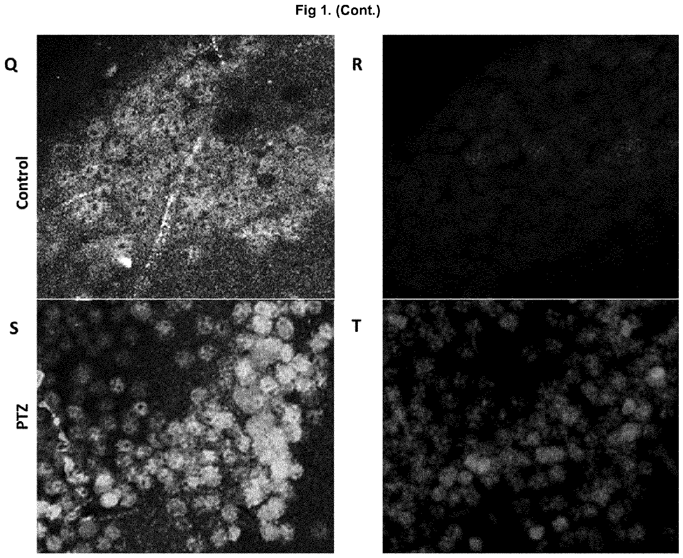

Figure 1. PTZ (Pentylenetretazole) induces activity of connexin hemichannels in brain cells. Representative images of the hippocampus in slices of brain. The different cell types in brain slices of control animals (two upper panels of each figure) or of animals treated with PTZ (two lower panels of each figure). The white zones correspond to the reactivity of each specific cellular marker (Panels to the left: A, C, E, G, I, K, M, O, Q and S) and ethidium uptake in cells of the same field from the right: B, D, F, H, J, L, N, P, R y T). Mast cells (A-B), microglia (C-D), astrocytes (E-F), oligodendrocytes (G-H) and neurons (I-J) from PTZ treated and control mice.
Figure 2. Effect of PTZ on the activation of hemichannels in the different cell types is time dependent.
Figure 3. Carbenoxolone, a non-selective blocker of PTZ-induced activity of hemichannels and gap junction channels, formed respectively by connexins and pannexins in macroglia and neurons.

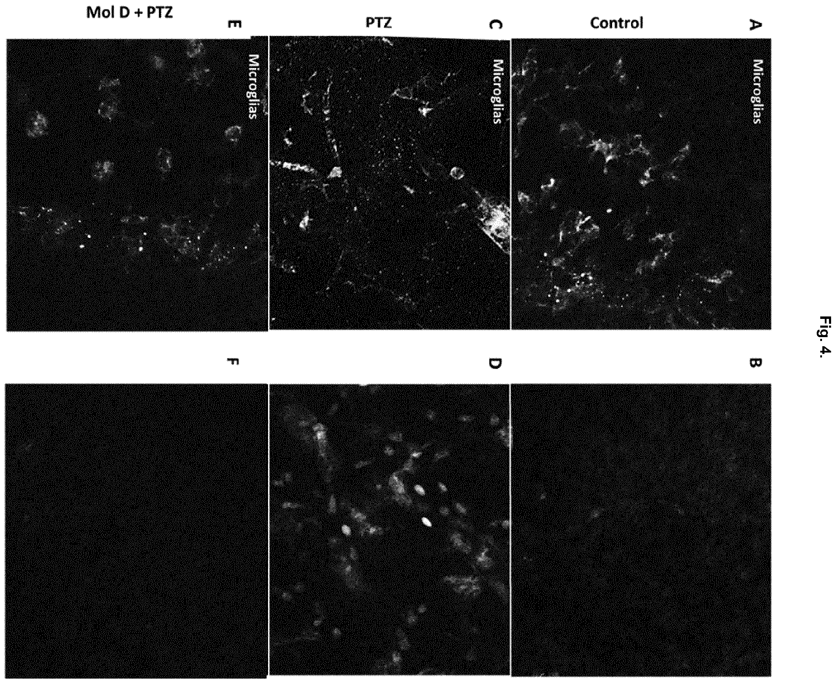

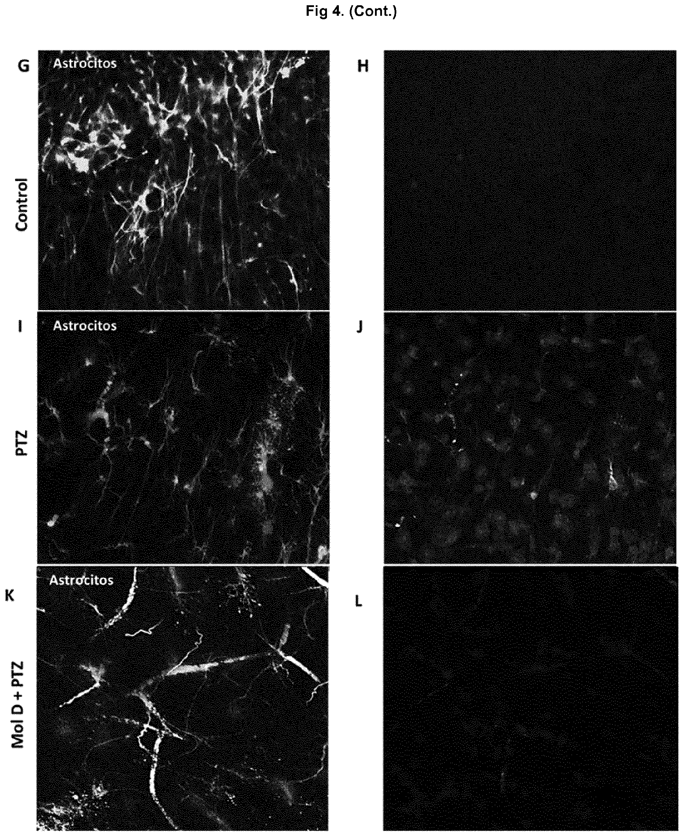

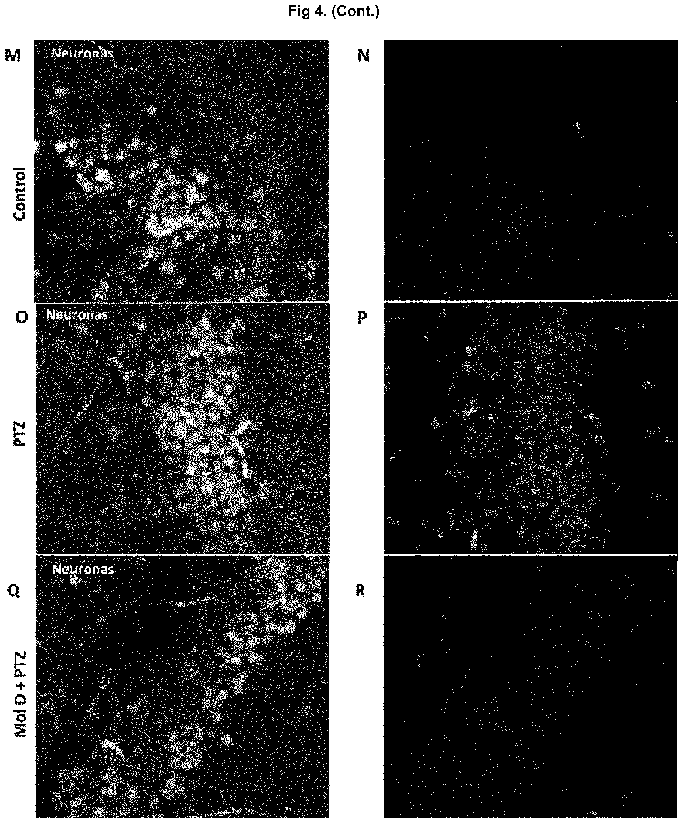

Figure 4. The selective connexin hemichannel blocker 2-(4-chlorophenyl)-2-oxo-1-phenylethyl quinoline-2-carboxylate (D4) blocks the activity of hemichannels formed by connexin PTZ-induced in brain cells. Representative images of hippocampus from brain slices. The different cell types are shown in white on the left panels: microglia (A, C, E), astrocytes (G, I, K) and neurons (M, O, K), of control mice, PTZ-induced and pre-treated with 2-(4-chlorophenyl)-2-oxo-1-phenylethyl quinoline-2-carboxylate (D4) and PTZ. Ethidium uptake is observed in white on the right panels for each cell type, respectively, of control mice (B, H, N), PTZ treated mice (D, J, P) and mice pretreated with 2-(4-chlorophenyl)-2-oxo-1-phenylethyl quinoline-2-carboxylate (D4) and then treated with PTZ (F, L, R).
Figure 5. The selective connexin hemichannel blocker 2-(4-chlorophenyl)-2-oxo-1-phenylethyl quinoline-2-carboxylate (D4) blocks PTZ-induced hemichannels activity in brain cells.

### BRIEF DESCRIPTION OF THE INVENTION

The present invention relates to hemichannel blocking compounds formed by connexins for the treatment of epilepsy.

The present invention further provides a composition comprising such compounds in combination with other antiepileptic compounds.

In a preferred embodiment, the present invention provides a composition of connexin hemichannels blockers with an antiepileptic or anticonvulsive compound, such as, for example, phenytoin for the total control of epileptic seizures.

### DETAILED DESCRIPTION OF THE INVENTION

In one embodiment of the invention, there are provided methods of using connexin hemichannel blockers for the elaboration of medicaments useful for treating epilepsy.

In another embodiment of the invention, there are provided methods of using connexin hemichannel blockers and the manufacture of medicaments useful for treating epilepsy in combination with compounds that block the sodium ion channels in the brain.

In another embodiment of the invention, there is provided a composition comprising a connexin hemichannel blocker in combination with phenytoin to treat epilepsy.

Unlike the presently used medications for the treatment of epilepsy, which correspond to drugs with action on molecular targets located in neurons, the present invention provides a treatment focused on glial cells, which are responsible for the inflammatory state triggered when neuronal activity exacerbates, and which under normal conditions nourish neurons and maintain the neurons environment for normal function. In contrast, if glia displays an increased hemichannel activity they cannot fulfill these normal functions, leaving the neurons in a state of deregulated excitation, which could lead to excitotoxicity or neuronal death. Therefore, the initial hypothesis to develop the present invention is that the increase of neuronal activity produces a high release of neurotransmitters and potassium ions that activate the glial cells (micro- and macroglia), that initiate an inflammatory state, which is perpetuated favoring the recurrence of increased neuronal activity.

Therefore, with a treatment focused on reducing or avoiding neuroinflammation by reducing the activity of connexin hemichannel in glial cells, the recurrence of high neuronal activity and excitoxicity is prevented.

Consistent with this mechanism, it has been demonstrated that in other conditions that trigger the neuroinflammation response, such as cerebral infarction and Alzheimer's disease, and using experimentally useful strategies, we demonstrate that the activity of glial hemichannels significantly increases and participates in cell degeneration. In cerebral infarction in the presence of high glycemia not only induces neuronal death but also glial cells death, and in vitro studies have shown that the inhibition of the hemichannels drastically reduces astroglial death in cell cultures exposed to high glucose and lack of oxygen, a condition that mimics cerebral infarction (Orellana, Hernández et al. Glia 2010 Feb; 58(3):329-43). In addition, we have demonstrated that the amyloid beta peptide, responsible for the neuroinflammation present in Alzheimer's disease, increases the activity of the glial hemichannels and therefore the cells release more glutamate and ATP inducing neuronal death (Orellana, Froger et al. Neurochem. 2011 Sep, 118(5):826-40, Orellana, Shoji et al. J. Neurosci. 2011 Mar 30, 31(13):4962-77).

Consequently, the use of connexin hemichannel blockers allows drastically reducing brain deterioration and thus substantially improves the quality of life of the patient.

To show the properties of the selective connexin hemichannel blocker 2-(4-chlorophenyl)-2-oxo-1-phenylethyl quinoline-2-carboxylate (D4) as an anticonvulsant drug, an acute epilepsy model was used in C57BL/6 mice, which is used to generate epileptic activity in a normal brain induced with a chemical seizure agent. PTZ (Pentylenetetrazole) was used as the epileptogenic agent and a dose response curve was determined to determine the effective dose, as shown in Example 1.

Optionally, it is possible to use other connexin hemichannel blockers, such as 1-benzyl 4-chlorophenyl-piperazine for the treatment of epilepsy.

In the last 5 years, changes in mRNA levels and expression of connexins and pannexins associated with epileptogenic processes have been described through different models of rodent epilepsy (Mylvaganam, Ramani et al. Front Physiol 2014 May 7; 5:172). The use of pannexin 1 (Panx1) knockout mice has shown that the activation of channels formed by Panx1 contributes to neuronal hyperactivity during the epileptogenic episode (Santiago, Veliskova et al. PLoS One 2011; 6(9):e25178). However, the contribution of glial cells during an epileptic seizure has not been studied. To determine the involvement of connexins in the different brain cell types over time, a time course was performed to measure hemichannel activity at 10, 20 and 30 min post PTZ administration by means of the dye uptake technique as shown in Example 2.

To determine that the activity of hemichannel in the macroglia and neurons is mediated by hemichannels and channels formed by connexins and pannexins, respectively, it was characterized by its sensitivity to carbenoxolone (Cbx), a non-selective blocker of both hemichannels and channels formed by connexins and pannexins, respectively) as shown in Example 3.

In PTZ-treated mice, this non-selective agonist compound of GABA_{A} receptors was shown to cause animals to develop seizures within 10 minutes post-administration, followed by a period of several hours where the mice exhibit very low motor activity and sporadic contractions. However, animals receiving the selective connexin hemichannel blocker 2-(4-chlorophenyl)-2-oxo-1-phenylethyl quinoline-2-carboxylate (D4) only exhibit seizures within 10 minutes after PTZ administration, but afterwards their behavior is similar to that of animals not treated with PTZ.

In addition, the application of PTZ to brain slices induced the opening of glial hemichannels.

### Advantage

In contrast to current epilepsy treatments focused on molecular targets located in neurons, the present invention focuses on glial cells. This is because the increase of the neuronal activity produces greater release of neurotransmitters and of potassium ions that activates the glia, inducing an inflammatory state that perpetuates an imbalance of the neuronal microenvironment favoring the recurrence of the increase of neuronal activity.

Therefore, if the treatment focuses on reducing or avoiding neuroinflammation, recurrences of epileptic seizures are prevented, substantially improving the quality of life of the patient.

In addition, the combined use of connexin blockers together with a current drug antiepileptic drug, allows epileptic seizures to be completely avoided, exceeding by far 70% in seizure control achieved by current drugs targeting sodium channels in brain. This would further improve the social inclusion and quality of life of patients with this condition.

As mentioned, 30% of epileptic patients do not respond to current drugs, so the use of a connexin hemichannel blocker as therapeutic agents results in a relevant solution in refractory cases.

Based on the development of the present invention, it was observed that phenytoin treatment does not prevent PTZ-induced seizures after 10 minutes of treatment. However, connexin blockers prevent them completely.

Therefore, as shown in the examples, the combined use of the selective connexin hemichannel blocker 2-(4-chlorophenyl)-2-oxo-1-phenylethyl quinoline-2-carboxylate (D4) and another antiepileptic agent allows the control of epileptic seizures completely and offers a solution in cases refractory to therapy.

According to the evaluated criteria, there were no observed side effects at the evaluated doses (see Example 4), where the score was zero for all the variables in each treatment studied. In addition, the mortality was zero at all concentrations evaluated, demonstrating the low risk of the use of said compound.

To determine the efficacy of the selective connexin hemichannel blocker 2-(4-chlorophenyl)-2-oxo-1-phenylethyl quinoline-2-carboxylate (D4) as anti-epileptic it was evaluated as a pretreatment to the induction of epilepsy with PTZ as shown in Example 5.

### EXAMPLES

### Example 1: Use of PTZ (Pentylenetetrazole) to induce epileptogenic episodes in C57BL /6 mice model.

As epileptogenic agent, we used PTZ (Pentylenetetrazole), a GABA_{A} receptor inhibitor widely used in the study and development of anticonvulsant drugs.

To determine the effective dose, a dose response curve was performed based on the available literature. Adult male mice of 2 months were separated in individual cages for intraperitoneal PTZ administration of a single dose of 65, 70, 75, 80 and 90 mg/kg of mouse weight. It was observed that the 70 mg/kg dose induces convulsions, from facial stereotypes (facial movements) to myoclonic seizures (kangaroo posture), clonic (overturned) and tonic-clonic (backward) seizures, without being lethal (Table 1).

Higher doses (> 100 mg/kg) induce epileptogenic episodes, but with a 100% mortality rate at 5 min after the administration of the seizure agent, whereas a lower dose (<65 mg/kg) does not induce evident epileptogenic episodes.

**Table 1. Dose Response of PTZ in adult C57BL/6 mice**

| Treatment | Eliptogenic Episode | Mortality | % Mortality |
|---|---|---|---|
| PTZ (90 mg/kg) | Yes | 3/3 | 100 |
| PTZ (80 mg/kg) | Yes | 3/3 | 100 |
| PTZ (75 mg/kg) | Yes | 2/4 | 50 |
| PTZ (70 mg/kg) | Yes | 3/15 | 20 |
| PTZ (65 mg/kg) | No | 0/3 | 0 |

### Example 2: Activation of hemichannels in PTZ-induced brain cells is time dependent.

Adult male mice of 2 months were injected via i.p. with PTZ (70 mg/kg) or phosphate buffered saline (PBS) as a control. Mice were sacrificed after 10, 20 and 30 min posttreatment to extract the brains and generate coronal slices of 400 µm thick using a vibratome. The slices were stabilized for 1 h prior to dye uptake, immersed in ACSF (artificial cerebrospinal fluid), bubbled with 95% O₂ and 5% CO₂ and at room temperature. Brain slices were incubated with the ethidium bromide dye (Etd, 4 µM) for 10 minutes. Subsequently, the slices were washed and fixed for 1 h with paraformaldehyde (4%). The brain slices were incubated for 40 min with blocking solution (PBS, 0.2% gelatine, 1 % triton). Subsequently, the samples were incubated overnight at 4 °C with the primary antibody against molecular markers of the different cell types: anti-mast cell "Transmembrane tyrosine kinase" receptor CD117 (CD117); Integrin αM anti-microglia (CD11b); anti-astrocytes "glial fibrillary acidic protein" (GFAP); anti-oligodendrocytes "proteolipid protein" (PLP) and anti-neuronal neuron-market (NeuN). After washing with PBS (phosphate buffered saline), they were incubated with the Cy2-conjugated secondary antibody for 2 hours at room temperature and avoiding light.

Finally, the brain slices were washed and mounted on Flouromount G mounting medium and observed directly by the laser-scanning confocal microscope (Nikon Eclipse Ti). Consecutive images were taken with a 40X lens at 1 µm intervals sequentially with two lasers (Argon, 488 nm and Helium/Neon, 543 nm). In Figure 1, in the hippocamp region, representative images of mast cells (1.A, C), microglia (1.E, F), astrocytes (1.1, K), oligodendrocytes (1.M, O) and neurons (1.Q, S) and Etd-uptake of control mice and PTZ-induced mice (1.B, D, 1.F, H, 1.J, L, 1.N, P, 1.R, T). The brain slices of animals treated with 70 mg/kg PTZ are positive for high activity of hemichannels (ethidium fluorescence seen as bright white which reveals high activity of hemichannels with respect to their controls.) Calibration bar = 10 µm.

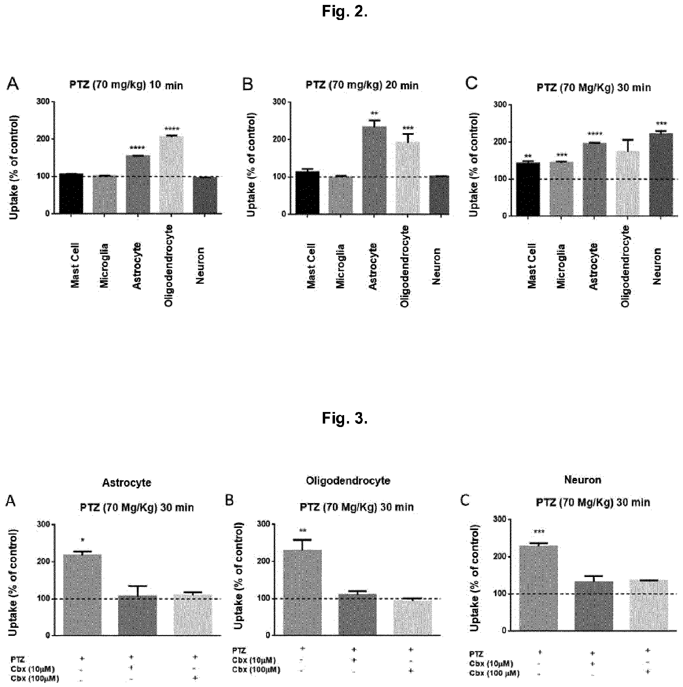

The mean data of ethidium uptake was normalized to the control condition (dotted line) of each cell type. The results show that PTZ sequentially increases the activity of the hemichannels first in astrocytes and oligodendrocytes (Figure 2.A), then after 30 min in mast cells, microglia and neurons (Figure 2.B and C). These results suggest that the activation of hemichannels in macroglia (astrocytes and oligodendrocytes) could trigger neuronal hyperactivity during the epileptogenic episode. The results obtained were expressed as the mean ± SEM. Samples were determined using the Student's t-test (* p <0.05).

### Example 3: Activation of hemichannels in PTZ-induced brain cells is blocked by carbenoxolone.

Adult male mice at 2 months were injected intraperitoneally with PTZ (70 mg/kg) or PBS as a control. After 30 min post injection of the treatment, the action of Cbx at two concentrations was evaluated, 10 µM to block pannexin hemichannels and 100 µM to block both connexin and pannexin-based channels. The mice were sacrificed to extract the brains and obtain coronal slices 400 µm thick. The slices, once stabilized, were treated with Cbx for 20 min and then incubated with the ethidium bromide dye (4 µM) for 10 minutes. Thereafter, the slices were fixed and the immunofluorescence staining process was continued as described above. The mean data of ethidium uptake was normalized to the control condition (dotted line) of each cell type. The results demonstrate that both concentrations of Cbx block the uptake of ethidium (activity of connexin hemichannels and pannexin channels, respectively) induced by PTZ at baseline in astrocytes (Figure 3A), oligodendrocytes (Figure 3B) and neurons (Figure 3C). These results suggest the participation of connexin hemichannels and pannexin channels activity in the epileptogenic episode. The results obtained were expressed as the mean ± SEM. Samples were determined using the Student's t-test (* p <0.05).

### Example 4: The selective connexin hemichannel blocker 2-(4-chlorophenyl)-2-oxo-1-phenylethyl quinoline-2-carboxylate (D4) does not affect viability in C57B / 6 mice

To evaluate the toxicity or lethality of the selective connexin hemichannel blocker 2-(4-chlorophenyl)-2-oxo-1-phenylethyl quinoline-2-carboxylate (D4), two-month adult mice were injected intraperitoneally in increasing doses (0.2; 2; 20; 200; 2000 mg/kg mouse weight) and were left under observation for 72 hours. After the observation time, the behavioral and physiological aspects of the animals were evaluated based on the Protocol of Supervision according to the Bioethics and Biosafety guidelines of the Faculty of Biological Sciences of the Pontificia Universidad Catolica de Chile that regulate the laboratory. The variables considered included physiological data (weight loss), appearance (posture) and spontaneous behavior (attitude to stimulation). According to the criteria evaluated, there were no observable side effects in any of the evaluated doses, the score was zero for all the variables in each treatment studied, considering that a score of 0-3 is normal (Table 2). In addition, mortality was zero at all concentrations evaluated. The evaluation of mortality of the selective connexin hemichannel blocker 2-(4-chlorophenyl)-2-oxo-1-phenylethyl quinoline-2-carboxylate (D4) as an anticonvulsant drug administered in mice prior to the induction of epilepsy with PTZ was zero (Table 3).

**Table 2: Supervision protocol for mice treated with 2-(4-chlorophenyl)-2-oxo-1-phenylethyl quinoline-2-carboxylate (D4).**

| Variables | | Periodicity | Score |
|---|---|---|---|
| PHYSIOLOGICAL DATA Weight Loss | Normal (no weight loss) | Daily | 0 |
| | Weight loss less than 10% | | 1 |
| | Weight loss between 10-20%. Possible decrease in food intake | | 2 |
| | Weight loss greater than 20%. The animal does not consume water or food | | 3 |
| ASPECT Posture | Normal (erect, hangs in its cage, glossy coat) | Daily | 0 |
| | Avoid moving, hair hirsute, there may be porphyric secretion in the nose and eyes (red) | | 1 |
| | Bent, immobile at the bottom of the cage, dull, hirsute fur, porphyritic discharge in the nose and eyes (red) | | 2 |
| | Prostrated, lateral cubit, evident dehydration, sunken eyes. | | 3 |
| SPONTANEOUS BEHAVIOR (Attitude towards stimuli) | Normal (attentive to the environment, interact with peers, grooming) | Daily | 0 |
| | Small changes (reduces grooming and movement, rapid breathing movement, tends to stay at the bottom of the cage) | | 1 |
| | Scrambling, inactive or retracted to the bottom of the cage, abdominal breathing. | | 2 |
| | Immobile animal, prostrate with lateral cubit, open mouth when breathing. | | 3 |

| | | | |
|---|---|---|---|
| Total Score from 0 to 12 Note: When an animal gets a score of 3 on more than one parameter, all "3" changes to "4". | | | |

Measurements are performed by trained personnel, who know the basal or usual behavior of the animals under study.

Corrective measures suggested according to the score obtained for each animal are the following:
- From 0-3 Normal
- From 4-6 Cautious Monitoring.
- From 7-12 Heavy Suffering

Endpoint Criteria: Scores between 7-12 should be indicators that the animal shows significant deterioration or severe suffering.

**Table 3. The selective connexin hemichannel blocker 2-(4-chlorophenyl)-2-oxo-1-phenylethyl quinoline-2-carboxylate (D4) as an anticonvulsant drug is not lethal.**

| Treatment | | | |
|---|---|---|---|
| 2-(4-chlorophenyl)-2-oxo-1-phenylethyl quinoline-2-carboxylate (D4) (2 mg/kg) | PTZ (70 mg/kg) | Animal Mortality | % Mortality |
| No | Yes | 3/15 | 20 |
| No | Yes | 0/3 | 0 |

### Example 5: The selective connexin hemichannel blocker 2-(4-chlorophenyl)-2-oxo-1-phenylethyl quinoline-2-carboxylate (D4) blocks PTZ-induced hemichannel activity in brain cells

Adult male mice of 2 months were injected intraperitoneally with 2 mg/kg of the selective connexin hemichannel blocker 2-(4-chlorophenyl)-2-oxo-1-phenylethyl quinoline-2-carboxylate (D4), in two instances: six and one hour before administration of PTZ (70 mg/kg). In the case of control animals, phosphate buffered saline (PBS) is used. After 30 min post-administration of PTZ the mice were sacrificed to extract the brains and subsequently measure the activity of the hemichannels with the technique of ethidium uptake. Immunofluorescence and visualization in confocal microscopy identified cell types.

Animals pretreated with the selective connexin hemichannel blocker 2-(4-chlorophenyl)-2-oxo-1-phenylethyl quinoline-2-carboxylate (D4) do not manifest convulsions and/or body contractures or behavioral changes evident unlike the control mice.

Figure 4 shows representative images of the hippocampal region where white (4.A, C, E), astrocytes (4.G, I, K) and neurons (4.M, O, Q) are shown. Etd uptake was observed in white on the right panels for each cell type, respectively, of control mice (4.B, H, N), mice treated with PTZ (4.D, J, P) and mice pretreated with the selective connexin hemichannel blocker 2-(4-chlorophenyl)-2-oxo-1-phenylethyl quinoline-2-carboxylate (D4) and then treated with PTZ (4.F, L, R). The brain slices of animals treated with 70 mg/kg PTZ are positive for Etd fluorescence, which reveals high activity of hemichannels with respect to their controls, whereas slices from mice pretreated with the selective connexin hemichannel blocker 2-(4-chlorophenyl)-2-oxo-1-phenylethyl quinoline-2-carboxylate (D4) are not positive to Etd as well as controls. Calibration bar = 10 µm.

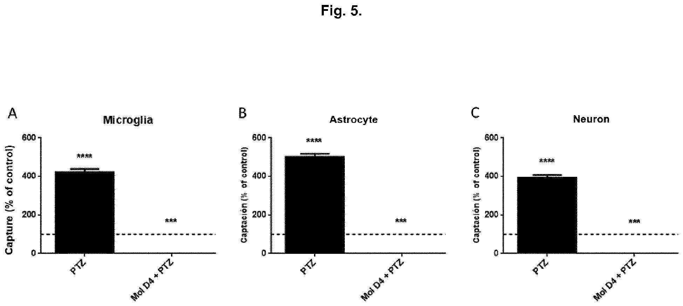

The mean data from ethidium uptake was normalized to the control condition (see Figure 5, dotted line) of each cell type. The results show that pre-treatment with the selective connexin hemichannel blocker 2-(4-chlorophenyl)-2-oxo-1-phenylethyl quinoline-2-carboxylate (D4)blocks PTZ-induced hemichannel activity and basal activation in microglia, astrocytes and neurons. These results indicate that the selective connexin hemichannel blocker 2-(4-chlorophenyl)-2-oxo-1-phenylethyl quinoline-2-carboxylate (D4) is an effective blocker of hemichannel activity during an epileptogenic episode. The results obtained were expressed as the mean ± SEM. Samples were determined using the Student's t-test (* p <0.05).

## Claims

1. Pharmaceutical composition for use in the treatment of epilepsy, **CHARACTERIZED in that** it comprises a pharmaceutically acceptable amount of a selective inhibitor compound of connexin hemichannels.

2. Pharmaceutical composition according to claim 1, **CHARACTERIZED in that** the selective inhibitor compound of connexin hemichannels is (D4) 2- (4-chlorophenyl) -2-oxo-1-phenylethyl 2-quinolinecarboxylate.

3. A synergic pharmaceutical composition, **CHARACTERIZED in that** it comprises as active compounds:
a) a selective inhibitor compound of connexin hemichannels; and
b) an antiepileptic compound inhibitor of sodium channels.

4. A pharmaceutical composition according to Claim 3, **CHARACTERIZED in that** compound a) is 2- (4-chlorophenyl)-2-oxo-1-phenylethyl 2-quinolinecarboxylate.

5. A pharmaceutical composition according to claim 3, **CHARACTERIZED in that** compound b) is selected from carbamazepine, valproate or phenytoin.

6. A pharmaceutical composition according to claim 3, **CHARACTERIZED in that** compound b) is preferably phenytoin.

7. A dosage form **CHARACTERIZED in that** comprises a composition according any of claims 1 to 6 and adjuvant agents.

8. A dosage form according to claim 7, **CHARACTERIZED in that** is suitable for oral, intravenous, intraarterial, intraperitoneal, intradermal, transdermal, intrathecal, intramuscular, intranasal, transmucosal, subcutaneous or rectal administration.

9. Use of selective inhibitor compounds of connexin hemichannels, **CHARACTERIZED in that** it is for the preparation of a medicament for the treatment of epilepsy.

10. Use according to claim 9, **CHARACTERIZED in that** the medicament additionally comprises one or more anti-epileptic compounds inhibitors of sodium channels.

11. Use according to claim 10, **CHARACTERIZED in that** the anti-epileptic compound inhibitor of sodium channel is phenytoin.
